(19) Europäisches Patentamt
European Patent Office
Office européen des brevets

(11) Veröffentlichungsnummer : **0 134 194**
**B1**

(12) # EUROPÄISCHE PATENTSCHRIFT

(45) Veröffentlichungstag der Patentschrift : **10.09.86**

(51) Int. Cl.⁴ : **C 07 C153/07**, C 09 K 15/14, C 08 K 5/36, C 10 L 1/24, C 10 M135/00

(21) Anmeldenummer : **84810361.0**

(22) Anmeldetag : **23.07.84**

(54) **S-(4-Hydroxyphenyl)-thioester.**

(30) Priorität : **29.07.83 US 518561**

(43) Veröffentlichungstag der Anmeldung : **13.03.85 Patentblatt 85/11**

(45) Bekanntmachung des Hinweises auf die Patenterteilung : **10.09.86 Patentblatt 86/37**

(84) Benannte Vertragsstaaten : **DE FR GB IT**

(56) Entgegenhaltungen :
**CHEMICAL ABSTRACTS, Band 82, Nr. 23, 9. Juni 1975, Seite 557, Nr. 155771u, Columbus, Ohio, USA**
**CHEMICAL ABSTRACTS, Band 67, Nr. 15, 9. Oktober 1967, Seite 6901, Nr. 73382u, Columbus, Ohio, USA**

(73) Patentinhaber : **CIBA-GEIGY AG**
**Klybeckstrasse 141**
**CH-4002 Basel (CH)**

(72) Erfinder : **Spivack, John D.**
**1 Blue Jay Street**
**Spring Valley New York 10977 (US)**
Erfinder : **Pastor, Stephen D.**
**112 Union Road**
**Spring Valley New York 10977 (US)**

**EP 0 134 194 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung bettrifft neue S-(4-Hydroxyphenyl)-thioester Derivate, deren Verwendung als Stabilisatoren sowie das damit stabilisierte organische Material.

Organische polymere Verbindungen wie Kunststoffe oder Harze unterliegen thermischem, oxidativem oder lichtinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welchem er eingesetzt wird, und vom jeweiligen Abbaumechanismus ab. Daher ist es im allgemeinen schwierig, für eine konkrete Anwendung den wirksamsten und ökonomischsten Stabilisator anzugeben.

So kann beispielsweise ein Stabilisator, der die Flüchtigkeit einer Verbindung reduziert, dahingehend wirken, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenbruch verhindert. Um die Vergilbung eines Substrats zu unterbinden, muss verhindert werden, dass Reaktionen des Substrats oder des Stabilisators ablaufen, die zu neuen Chromophoren führen. Weiterhin müssen Probleme der Verarbeitungsstabilität und der Substratverträglichkeit beachtet werden.

Ueberraschenderweise ist jetzt gefunden worden, dass spezielle Thioesterderivate eine ungewöhnliche Kombination wünschenswerter Eigenschaften besitzen, wodurch diese Stoffe zu besonders effektiven und nützlichen Stabilisatoren werden. Die erfindungsgemässen Verbindungen erweisen sich als besonders nützliche Stabilisatoren für Polyolefine, schlagfestes Polystyrol, Elastomere wie Polybutadien oder Styrol-Butadien-Elastomere oder andere Elastomere, bei denen der Erhalt der Elastizität, die Verhinderung von Vernetzungsreaktionen von Verfärbung, von Geruchsbildung und von Ausschwitzen des Stabilisators grundlegende Anforderungen an die Qualität eines Stabilisators darstellen.

Merkaptophenolderivate wurden bereits beschrieben. Viele dieser Verbindungen sind jedoch Hydroxyphenylthioalkanoatester.

Thioester werden in JP-Kokai *67,* 6 332 und in JP-Kokai *74,* 116 036 als Antioxidantien beschrieben.

Die vorliegende Erfindung betrifft Verbindungen der Formel I

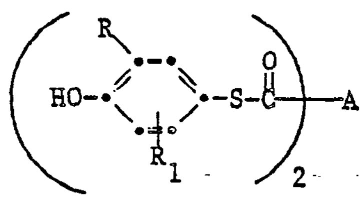

(I)

worin R und $R_1$ unabhängig voneinander Wasserstoff, $C_1$-$C_{12}$Alkyl, $C_5$-$C_6$Cycloalkyl, Phenyl, durch $C_1$-$C_{12}$Alkyl substituiertes Phenyl, $C_7$-$C_9$Aralkyl oder durch $C_1$-$C_{12}$Alkyl substituiertes $C_7$-$C_9$Aralkyl bedeuten, und worin A $C_1$-$C_{10}$Alkylen, $C_5$-$C_6$Cycloalkylen, Arylen, Biphenylen oder hydroxysubstituiertes Arylen bedeutet.

Die Reste R und $R_1$ bedeuten bevorzugt geradkettiges oder verzweigtes $C_1$-$C_8$Alkyl, wie beispielsweise Methyl, n-Butyl, sec.-Butyl, tert.-Butyl, tert.-Pentyl, 2-Ethylhexyl, n-Octyl oder 1,1,3,3-Tetramethylbutyl. Besonders bevorzugt werden die Reste Methyl, tert.-Butyl, tert.-Pentyl und 1,1,3,3-Tetramethylbutyl.

Als $C_5$-$C_6$-Cycloalkyl bedeuten R oder $R_1$ Cyclopentyl oder Cyclohexyl, bevorzugt Cyclohexyl.

Besonders bevorzugt werden Verbindungen der Formel I, worin $R_1$ sich in ortho-Position zur Hydroxygruppe befindet und tert.-Alkyl bedeutet, oder worin $R_1$ und R tert.-Butyl sind.

Ebenfalls von Interesse sind Verbindung der Formel I, worin $R_1$ sich in ortho-Position zur Hydroxygruppe befindet.

R und $R_1$ als Aralkyl bedeuten beispielsweise Benzyl, α-Methylbenzyl oder α,α-Dimethylbenzyl. Als durch $C_1$-$C_{12}$-Alkyl substituiertes Phenyl bedeuten R und $R_1$ beispielsweise Tolyl, Mesityl oder Xylyl.

Als $C_1$-$C_{10}$ Alkylen bedeutet A beispielsweise Methylen, Ethylen, Trimethylen, Tetramethylen, Pentamethylen, Hexamethylen, Octamethylen oder Decamethylen.

Als Arylen bedeutet A vorzugsweise o-, m- oder p-Phenylen.

Bevorzugte Verbindungen der Formel I sind diejenigen, bei denen A $C_1$-$C_{10}$Alkylen, bevorzugt $C_4$-$C_8$Alkylen, und besonders bevorzugt $C_8$Alkylen bedeutet, oder bei denen A Phenylen ist, und worin R und $R_1$ tert.-Butyl sind.

Die erfindungsgemässen Verbindungen lassen sich durch Umsetzen eines geeigneten Merkaptophenols mit einem Carbonsäurechlorid in Gegenwart eines Protonenakzeptors herstellen. Typische Protonenakzeptoren sind beispielsweise Lithiumsalze, tert.-Amine, Alkalimetalle, Alkalimetall- und Erdalkalimetallhydroxide, Carbonate, u. v. a.

Die Reaktion wird in Lösung durchgeführt. Als Lösungsmittel eignen sich beispielsweise aromatische Kohlenwasserstoffe wie Benzol, Toluol, Xylol oder dergl. oder aliphatische Kohlenwasserstoffe wie Heptan. Die Reaktion wird im Temperaturbereich von 0 bis 70 °C durchgeführt. Die Ausgangsmaterialien zur Herstellung der erfindungsgemässen Verbindungen sind im Handel erhältlich oder sind nach bekannten Verfahren herstellbar.

. Die erfindungsgemässen Verbindungen lassen sich als Stabilisatoren für organisches Material wie beispielsweise Kunststoffe, Elastomere oder Harze verwenden ; sie lassen sich darüber hinaus als Stabilisatoren für Mineralöle oder synthetische Oele beispielsweise für Schmiermittel oder Wärmeaustauscheröle einsetzen.

Die Erfindung betrifft daher auch eine Zusammensetzung enthaltend ein gegen oxidativen, thermischen oder strahlungsinduzierten Abbau empfindliches organisches Material und mindestens eine Verbindung der Formel I als Stabilisator, wobei als organisches Material vorzugsweise ein Polymer oder ein Schmieröl zu verstehen ist, besonders bevorzugt aber ein polyolefinisches Homo- oder Copolymer, insbesondere aber auch ein Homo-, Co- oder Terpolymer von Styrol, oder ein Schmieröl, das sich vom Mineralöl ableitet.

Die Verbindungen der Formel I lassen sich insbesondere zur Stabilisierung der Homo- oder der Copolymeren von Polyolefinen einsetzen. Folgende polyolefinische Substrate lassen sich vorzugsweise mit den erfindungsgemässen Substanzen stabilisieren : Polyethylen, Polypropylen, Polyisobutylen, Poly-(buten-1), Poly-(penten-1), Poly-3(methylbuten-1), Poly-(4-methylpenten-1), unterschiedliche Ethylen-Propylen-Copolymere, EPM, EPDM und ähnliche Verbindungen, Polystyrol und seine Co- oder Terpolymeren, wie beispielsweise schlagfestes Polystyrol, ABS-Harze, SBR, Polyisopren, Polybutadien, Nitilkautschuk, Naturkautschuk, oder Polyester wie beispielsweise Polyethylenterephthalat, Polybutylenterephthalat und deren Copolymere.

Ebenso lassen sich Polyurethane, Polycarbonate oder Polyamide wie beispielsweise Nylon 6, Nylon 6/6 oder dergleichen, sowie deren Copolymere oder Polysulfone mit den erfindungsgemässen Verbindungen stabilisieren.

Ebenfalls bevorzugt werden Zusammensetzungen, worin das organische Material ein synthetischer Schmierstoff oder ein Schmierstoff auf Mineralölbasis ist.

Die in Frage kommenden Schmierstoffe sind dem Fachmann geläufig und z. B. im « Schmiermittel Taschenbuch (Hüthig Verlag, Heidelberg, 1974) » beschrieben.

Generell kann man die Stabilisatoren für folgende polymere Substrate verwenden :

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z. B. von Cyclopenten oder Norbornen.

2. Mischungen der unter 1) genannten Polymeren, z. B. Mischungen von Polypropylen mit Polyisobutylen.

3. Copolymere von Mono- und Diolefinen untereinander oder mit anderen Vinylmonomeren, wie z. B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z. B. Styrol-Butadien, Styrol-Acrylnitril, Styrol-Alkylmethacrylat, Styrol-Maleinanhydrid, Styrol-Acrylnitril-Methylacrylat ; Mischungen von hoher Schlagzähigkeit aus Styrol-Copolymeren und einem anderen Polymer wie z. B. einem Polyacrylat, einem Dien-Polymeren oder einem Ethylen-Propylen-Dien-Terpolymeren ; sowie Block-Copolymere des Styrols, wie z. B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z. B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z. B. als sogenannte ABS, MBS, ASA oder AES-Polymere bekannt sind.

7. Halogenhaltige Polymere, wie z. B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z. B. Polyvinylchlorid, Polyvinylidenchlorid, Polyvinylfluorid, Polyvinylidenfluorid ; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z. B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd,

Polypropylen-oxyd oder deren Copolymere mit Bisglycidylethern.

12. Polyacetale, wie Polyoxymethylen, sowie solche Polyoxymethylene, die Comonomere wie z. B. Ethylenoxid enthalten.

13. Polyphenylenoxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Block-Copolymere mit Polyethern wie z. B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, eie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige,schwerbrennbare Modifikationen.

23. Vernetzbare Acrylharze, die sich von substituierten Acrylsäureestern ableiten wie z. B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten, oder Epoxidharzen vernetzt sind.

25. Vernetzte Epoxidharze, die sich von Polyepoxiden ableiten, z. B. von Bis-glycidylethern, von cycloaliphatischen Diepoxiden oder von aromatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z. B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z. B. Phthalate, Adipate, Phosphate oder Trimellithate), sowie Abmischungen synthetischer Ester mit Mineralölen in beliebigen Gewichtsverhältnissen, wie sie z. B. als Spinnpräparationen Anwendung finden, sowie deren wässrigen Emulsionen.

29. Wässrige Emulsionen natürlicher oder synthetischer Kautschuke, wie z. B. Naturkautschuk-Latex oder Latices von carboxylierten Styrol-Butadien-Copolymeren.

Die Erfindung betrifft weiterhin ein Verfahren zum Stabilisieren von organischem Material gegen oxidativen, thermisch- oder strahlungsinduzierten Abbau, dadurch gekennzeichnet, dass man dem Material mindestens eine Verbindung der Formel I wie oben beschrieben zusetzt.

Ein weiterer Gegenstand der Erfindung ist die Verwendung von Verbindungen der Formel I als Stabilisatoren für organisches Material gegen dessen Schädigung durch Einwirkung von Sauerstoff, Wärme, Licht und energiereiche Strahlung.

Die erfindungsgemässen Stabilisatoren werden den Kunststoffen in Mengen von 0,01 bis 5 Gew.% bezogen auf das stabilisierte Material zugemischt. Je nach Verwendung und Substrat wird die Stabilisatormenge variiert. Bevorzugt verwendet man 0,05 bis 2 Gew.% des Stabilisators, besonders bevorzugt 0,1 bis 1 Gew.%.

Die Einarbeitung kann beispielsweise durch Einmischen der Substanzen der Formel I und gegebenenfalls weiterer Additive nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels erfolgen. Die neuen Verbindungen können auch in Form eines Masterbatches, der diese Verbindungen beispielsweise in einer Konzentration von 2,5 bis 25 Gew.-% enthält, den zu stabilisierenden Kunststoffen zugesetzt werden.

Im Falle von vernetztem Polyethylen werden die Verbindungen vor der Vernetzung beigefügt.

Die so stabilisierten Materialien können in verschiedenster Form angewendet werden, z. B. als Folien, Fasern, Bändchen, Formmassen, Profile oder als Bindemittel für Lacke, Klebstoffe oder Kitte.

In der Praxis können die Verbindungen der Formel I zusammen mit anderen Stabilisatoren eingesetzt werden.

Schmierstoff-Formulierungen können zusätzlich noch andere Additive enthalten, die zugegeben werden, um gewisse Gebrauchseigenschaften zu verbessern, wie z. B. weitere aminische Antioxidantien, Metallpassivatoren, Rostinhibitoren, Viskositäts-Index-Verbesserer, Stockpunkterniedriger, Dispergiermittel/Tenside und Verschleissschutzadditive.

Als weitere Additive, mit denen zusammen die erfindungsgemäss verwendeten Stabilisatoren eingesetzt werden können, sind beispielsweise zu nennen :

1. Antioxidantien

1.1. Alkylierte Monophenole

2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone

2,6-Di-tert.butyl-4-methoxyphenol
2,5-Di-tert.butyl-hydrochinon
2,5-Di-tert.amyl-hydrochinon
2,6-Diphenyl-4-octadecyloxyphenol
2,5-Di-tert.butyl-hydrochinon
3,5-Di-tert.butyl-4-hydroxyamid
Tris-(3,5-di-tert.butyl-4-hydroxyphenyl)-phosphit
3,5-Di-tert.butyl-4-hydroxyphenylstearat
Bis-(3,5-di-tert.butyl-4-hydroxyphenyl)-adipat

1.3 Hydroxylierte Thiodiphenylether

2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)
4,4'-Thio-bis-(3,6-di-sek.amylphenol)
4,4'-Bis-(2,6-dimethyl-4-hydroxyphenyl)-disulfid

1.4. Alkyliden-Bisphenole

2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.
1,1-Bis-(3,5-dimethyl-2-hydroxyphenyl)-butan

2,2-Bis-(3,5-di-tert.butyl-4-hydroxyphenyl)-propan
1,1,5,5-Tetra-(5-tert.butyl-4-hydroxy-2-methylphenyl)-pentan

1.5. Benzylverbindungen

1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz
1,4-Bis-(3,5-di-tert.butyl-4-hydroxybenzyl)-2,3,5,6-tetramethylbenzol
2,4,6-Tris-(3,5-di-tert.butyl-4-hydroxybenzyl)-phenol.

1.6. Acylaminophenole

4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.7 Ester der β-(3,5-Di-tert.butyl-4-hydroxyphenyl)-propionsäure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |
| Ethanol | 1,9-Nonandiol |
| Ethylenglykol | 1,2-Propandiol |
| Trimethylhexandiol | 3-Thia-pentadecanol |
| Trimethylolpropan | Trimethylolethan |
| 4-Hydroxymethyl-1-phospha-2,6,7-trioxabicyclo-[2,2,2]-octan | |

1.8. Ester der β-(5-tert.butyl-4-hydroxy-3-methylphenyl)-propionsäure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |
| Ethanol | 1,9-Nonandiol |
| Ethylenglycol | 1,2-Propandiol |
| 3-Thia-undecanol | 3-Thia-pentadecanol |
| Trimethylolpropan | Trimethylolethan |
| 4-Hydroxy-1-phospha-2,6,7-trioxabicyclo-[2,2,2]-octan | |

1.9. Amide der β-(3,5-Di-tert.butyl-4-hydroxyphenyl)-propionsäure, wie z. B.

N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin
N,N'-Di-(3,5-di-tert.butyl-4-hydroxyphenylpropionyl)-hexamethylendiamin
N,N'-Bis-(3,5-di-tert.butyl-4-hydroxyphenylpropionyl)-hydrazin

Im Folgenden sind Beispiele von weiteren Additiven aufgezählt, die zusammen mit den oben erwähnten Antioxidantien und den erfindungsgemässen Stabilisatoren eingesetzt werden können. Zu diesen Stoffen zählen beispielsweise :

2. UV-Absorber und Lichtschutzmittel

2.1. 2-(2'-Hydroxyphenyl)-benztriazole, wie z. B. das 5'-Methyl-, 3',5'-Di-tert.butyl-, 5'-Tert.butyl-, 5'-(1,1,3,3-Tetramethyl-butyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-, 3'-Methylbenzyl-5'-methyl-5-chlor-,4'-Hydroxy-, 4'-Methoxy-, 3'-Methyl-5'-carbomethoxy-ethyl-, 3',5'-Bis-(α,α-dimethylbenzyl)-5-chlor-, 3',5'-Di-tert.octyl-phenyl, 3',5'-Di-tert.-octylphenyl-5-chlor- oder 5-Chlor-3',5'-di-tert.amyl-Derivate.

2.2. 2-Hydroxybenzophenone, wie z. B. das 4-Hydroxy-, 4-Methoxy-, 4-Octoxy-, 4-Decyloxy-, 4-Dodecyloxy-, 4-Benzyloxy-, 4,2',4'-Tri-hydroxy-, 2'-Hydroxy-4,4'-dimethoxy-Derivat.

2.3 Ester von gegebenenfalls substituierten Benzoesäuren, wie z. B. 4-Tert.butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester oder der n-Octadecylester oder der 2-Methyl-4,6-di-tert.butylester.

2.4. Acrylate, wie z. B. α-Cyan-β,β-diphenylacrylsäure-ethylester bzw.-isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z. B. Nickelkomplexe des 2,2'-Thio-bis-[4-(1,1,3,3-tetramethylbutyl)-phenols], wie der 1 : 1- oder der 1 : 2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.butylbenzyl-phosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z. B.

Bis-(2,2,6,6-tetramethylpiperidyl)-sebacat
Bis-(1,2,2,6,6-pentamethylpiperidyl)-sebacat
n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-Tetramethyl-4-hydroxypiperidin und Bernsteinsäure, Kondensationsprodukt aus N,N'-(2,2,6,6-Tetramethylpiperidyl)-hexamethylendiamin und 4-tert.octylamino-2,6-dichlor-1,3,5-s-triazin,
Tris-(2,2,6,6-tetramethylpiperidyl)-nitrilotriacetat,
Tetrakis-(2,2,6,6-tetramethyl-4-piperidyl)-1,2,3,4-butantetracarbonsäure,
1,1'-(1,2-ethandiyl)-bis-(3,3,5,5-tetramethyl-piperazinon)
4-Benzoyl-2,2,6,6-tetramethylpiperidin,
4-Stearyl-oxy-2,2,6,6-tetramethylpiperidin,
3-n-Octyl-7,7,9,9-tetramethyl-1,3,8-triaza-spiro[4,5]decan-2,4-dion.

2.7. Oxalsäurediamide, wie z. B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho-und para-Methoxy- sowie von o- und p-Ethoxy-di-substituierte Oxaniliden.

3. Metalldesaktivatoren, wie z. B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z. B. Triphenylphosphit, Diphenyl-alkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit,Tristearyl-sorbit-triphosphit, Tetrakis-(2,4-di-tert.butylphenyl)-4,4'-biphenylen-diphosphonit, 3,9-Isodecyloxy-2,4,8,10-tetraza-3,9-diphospha-spiro-[5,5]-undecan und Tri-(4-hydroxy-3,5-di-tert.butylphenyl)-phosphit.

5. Peroxidzerstörende Verbindungen, wie z. B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z. B. Kupfersalze in Kombination mit Iodiden und/oder Phosphorverbindungen und Salze des zweiwertigen Mangans.

7. Basische Co-Stabilisatoren, wie z. B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höhere Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z. B. 4-tert.Butylbenzoesäure, Adipinsäure, Diphenylessigsäure.

9. Füllstoffe und Verstärkungsmittel, wie z. B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z. B. Weichmacher, Gleitmittel, Emulgatoren, Pigmente, Optische Aufheller, Flammschutzmittel, Antistatika, Treibmittel und Thiosynergisten wie beispielsweise Dilaurylthiodipropionat oder Distearylthiodipropionat.

11. Aminische Antioxidantien :

N,N'-Di-isopropyl-p-phenylendiamin
N,N'-Di-sec.-butyl-p-phenylendiamin
N,N'-Bis(1,4-dimethyl-pentyl)-p-phenylendiamin
N,N'-Bis(1-ethyl-3-methyl-pentyl)-p-phenylendiamin
N,N'-Bis(1-methyl-heptyl)-p-phenylendiamin
N,N'-Dicyclohexyl-p-phenylendiamin
N,N'-Diphenyl-p-phenylendiamin
N,N'-Di-(naphthyl-2-)-p-phenylendiamin
N-Isopropyl-N'-phenyl-p-phenylendiamin
N-(1,3-Dimethyl-butyl)-N'-phenyl-p-phenylendiamin
N-(1-Methyl-heptyl)-N'-phenyl-p-phenylendiamin
N-Cyclohexyl-N'-phenyl-p-phenylendiamin
4-(p-Toluol-sulfonamido)-diphenylamin
N,N'-Dimethyl-N,N'-di-sec.-butyl-p-phenylendiamin
Diphenylamin
4-Isopropoxy-diphenylamin
N-Phenyl-1-naphthylamin
N-Phenyl-2-naphthylamin
octyliertes Diphenylamin
4-n-Butylaminophenol
4-Butyrylamino-phenol
4-Nonanoylamino-phenol
4-Dodecanoylamino-phenol
4-Octadecanoylamino-phenol
Di-(4-methoxy-phenyl)-amin
2,6-Di-tert.-butyl-4-dimethylamino-methyl-phenol
2,4'-Diamino-diphenylmethan
4,4'-Diamino-diphenylmethan
N,N,N,N'-Tetramethyl-4,4'-diamino-diphenylmethan
1,2-Di-(phenylamino)-ethan
1,2-Di-[(2-methyl-phenyl)-amino]-ethan
1,3-Di-(phenylamino)-propan
(o-Tolyl)-biguanid
Di-[4-(1',3'-dimethyl-butyl)-phenyl]amin.

12. Metallpassivatoren :

für Kupfer, z. B. :
Benztriazol, Tetrahydrobenztriazol, 2-Mercaptobenzthiazol, 2,5-Dimercaptothiadiazol, Salicylidenpropylendiamin, Salze von Salicylaminoguanidin.

13. Rost-Inhibitoren :

a) Organische Säuren, ihre Ester, Metallsalze und Anhydride, z. B. :
N-Oleoyl-sarcosin, Sorbitan-mono-oleat, Blei-naphthenat, Dodecenylbernsteinsäure-anhydrid, Alkenylbernsteinsäure-Halbester, 4-Nonylphenoxy-essigsäure.

b) Stickstoffhaltige Verbindungen, z. B. :

I. Primäre, sekundäre oder tertiäre aliphatische oder cycloaliphatische Amine und Amin-Salze von organischen und anorganischen Säuren, z. B. öllösliche Alkylammoniumcarboxylate.

II. Heterocyclische Verbindungen z. B.:

Substituierte Imidazoline und Oxazoline.

c) Phosphorhaltige Verbindungen, z. B.:
Aminsalze von Phosphorsäurepartialestern.
d) Schwefelhaltige Verbindungen, z. B.:
Barium-dinonylnaphthalin-sulfonate, Calciumpetroleum-sulfonate.

14. Viskositätsindex-Verbesserer:

Polymethacrylate, Vinylpyrrolidon/Methacrylat-Copolymere, Polybutene, Olefin-Copolymere, Styrol/Acrylat-Copolymere.

15. Stockpunktniedriger:

Polymethacrylat, alkylierte Naphthalinderivate.

16. Dispergiermittel/Tenside:

Polybutenylbernsteinsäure-imide, Polybutenylphosphonsäurederivate, basische Magnesium-, Calcium-, und Bariumsulfonate und -phenolate.

17. Verschleissschutz-Additive:

Schwefel und/oder Phosphor und/oder Halogen enthaltende Verbindungen, wie geschwefelte pflanzliche Oele, Zinkdialkyldithiophosphate, Tritolyl-phosphat, chlorierte Paraffine, Alkyl- und Aryldisulfide.

## Beispiel 1

Herstellung von S,S'-Bis-(3,5-di-tert.-butyl-4-hydroxyphenyl)-dithioadipat.

In einem flammengetrockneten Kolben wird unter Stickstoff eine Lösung aus 3,84 g (0,021 Mol) Adipoylchlorid, 50 ml Toluol und 50 ml Heptan hergestellt. Die Lösung wird im Eisbad gekühlt. Anschliessend tropft man eine Lösung von 10,0 g (0,042 Mol) 2,6-Di-tert.-butyl-4-merkaptophenol, 4,25 g (0,042 Mol) Triethylamin in 50 ml Toluol und 50 ml Heptan dazu. Nach beendeter Zugabe wird die erhaltene Suspension auf Raumtemperatur erwärmen gelassen und solange gerührt, bis das ursprünglich eingesetzte Merkaptan umgesetzt ist (dünnschichtchromatographisch ermittelt). Die Reaktionsmischung wird anschliessend filtriert und der Filterrückstand zweimal mit je 50 ml warmem Toluol gewaschen. Die vereinigten Filtrate werden im Vakuum konzentriert und der Rückstand aus Acetonitril umkristallisiert. Man erhält 9,8 g (Ausbeute 80 %) weisser Kristalle vom F.P. 140 °C-143 °C.

| Analyse: | | | |
|---|---|---|---|
| berechnet: | C 69,6 | H 8,6 | S 10,9 |
| gefunden: | C 69,9 | H 9,0 | S 11,1 |

## Beispiel 2

Herstellung von S,S'-Bis-(3,5-di-tert.-butyl-4-hydroxyphenyl)-dithiosebacat.

Das in Beispiel 1 beschriebene Verfahren wird wiederholt, indem man 5,02 g (0,021 Mol) Sebacoylchlorid, 10,0 g (0,042 Mol) 2,6-Di-tert.-butyl-4-merkaptophenol und 4,25 g (0,042 Mol) Triethylamin in 100 ml Toluol und 100 ml Heptan miteinander umsetzt. Nach dem Umkristallisieren aus Acetonitril erhält man 6,2 g (Ausbeute 46 %) eines weissen Pulvers vom F.P. 82 °C-85 °C.

| Analyse: | | | |
|---|---|---|---|
| berechnet: | C 71,0 | H 9,1 | S 10,0 |
| gefunden: | C 70,8 | H 9,1 | S 9,8 |

## Beispiel 3

Herstellung von S,S'-Bis-(3,5-di-tert.-butyl-4-hydroxyphenyl)-dithioterephthalat.

Das in Beispiel 1 beschriebene Verfahren wird wiederholt, indem man 4,26 g (0,021 Mol) Terephthaloylchlorid, 10,0 g (0,042 Mol) 2,6-Di-tert.-butyl-4-merkaptophenol und 4,25 g (0,042 Mol) Triethylamin in 400 ml Toluol miteinander umsetzt. Nach Umkristallisieren des Rückstandes aus Methylethylketon erhält man 10,0 g (Ausbeute 78 %) weisse Kristalle vom F.P. 310 °C.

Analyse :
berechnet : C 71,3 H 7,6 S 10,6
gefunden : C 71,3 H 7,8 S 10,7

Beispiel 4

In diesem Beispiel wird die stabilisierende Wirkung der erfindungsgemässen Verbindungen in schlagfestem Polystyrol demonstriert.

a) Herstellung der Proben : Man stellt eine Lösung von 8 Gew.-% Polybutadien-Kautschuk (Firestone DIENE® 55) in monomerem Styrol her, indem man beide Komponenten in einer Quetschmühle mischt. Gleichzeitig wird in diesem Arbeitsgang 0,1 Gew.-% des zu testenden Stabilisators zu der Lösung gegeben. Schliesslich fügt man noch 500 ppm Zn-Stearat hinzu, um später die polymerisierte Probe leicht aus dem Reaktionsgefäss ablösen zu können. Die Polymerisation wird in einem Gefäss durchgeführt, das mit einem Wendelrührer ausgerüstet ist. Da die meisten IPS Blockpolymerisationen thermisch initiiert werden, wird in der vorliegenden Laboratoriumsversion kein Starter zugesetzt. Die Reaktion wird unter Stickstoff und unter Erwärmen durchgeführt. Der Reaktor wird innerhalb einer halben Stunde auf 121 °C erwärmt, und diese Temperatur wird aufrecht erhalten, bis zwischen 30 und 35 % des Monomeren umgesetzt sind (Zeitdauer ca. 2,5 Stunden). Während der Polymerisation wird heftig gerührt, und die Rührgeschwindigkeit wird so eingestellt, dass Polymerpartikel von 2 bis 4 µm Grösse entstehen. Anschliessend werden die Reaktionsgefässe dem Reaktor entnommen, im Stickstoffstrom geöffnet und in ein Wirbelschichtsandbad gestellt, um die Polymerisation zu vervollständigen. Die Gefässe werden im Standbad folgendermassen erhitzt : eine Stunde bei 100 °C um die Anfangstemperatur vorzugeben, eine weitere Stunde, um 140 °C zu erreichen und anschliessend weitere 8 Stunden, wobei die Temperatur jede Stunde um 10 °C erhöht wird, bis schliesslich ein Maximum von 220 °C erreicht ist. Nach dem Abkühlen werden die Polymerisationsgefässe zerbrochen und das Glas entfernt. Ein einziger Polymerblock wiegt im Mittel etwas über 600 g. Der Block wird in einem Vakuumofen bei 200 °C für 45 Minuten bei einem Druck von 1,33 mbar belassen, um flüchtige Bestandteile zu entfernen. Nach dieser Behandlung wird der Block sofort in einer heizbaren hydraulischen Presse bei 205 °C zwischen zwei Aluminiumfolien zu einem dicken Stab gepresst (drei Minuten Erhitzen, fünf Minuten ungeheizt). Der Stab wird mittels einer Bandsäge zerkleinert und anschliessend granuliert. Alle Polymermischungen werden bei 205 °C extrudiert und dann zerkleinert. Die Pellets werden unter Druck bei 205 °C in dehnbare 3,2 mm dicke Streifen verformt.

b) Testverfahren : Diese Probestreifen werden dann bei 150 °C in einem Umluftofen gealtert, wobei sie auf Glasplatten, die sich wiederum auf rotierenden Trägern befinden, aufgebracht sind. Eine weitere Charge von Probestreifen wird auf dieselbe Weise bei 80 °C im Ofen gealtert. Nach bestimmten Zeitintervallen wird der Yellowness Index der Proben gemäss ASTM D-1925-63T bestimmt, und es wird die Dehnbarkeit gemäss ASTM D638 bestimmt (Instron Tensile Testing Apparatus, Instron Engineering Corporation, Massachusetts ; Ziehgeschwindigkeit : 5 mm/Minute).

In der folgenden Tabelle 1a sind die Ergebnisse der Dehnbarkeitsmessungen und der Messungen des Yellowness Indexes von bei 80 °C ofengealterten Proben angegeben, während Tabelle 1b Ergebnisse von bei 150 °C ofengealterten Proben enthält.

Tabelle 1a

Dehnbarkeitsmessungen und Yellowness Index von bei 80 °C ofengealterten Proben mit und ohne Stabilisator

| Additiv | Dehnbarkeit der Probe (%) und Dauer der Hitzebelastung (h) | | | | |
|---|---|---|---|---|---|
| | 0 h | 300 h | 600 h | 900 h | 1200 h |
| Produkt von Beispiel 2 | 50 | 22 | 10 | 6 | 4 |
| – | 33 | 9 | 3 | 3 | 3 |

(Fortsetzung)

| Additiv | Yellowness Index und Dauer der Hitzebelastung (h) | | | | |
|---|---|---|---|---|---|
| | 0 h | 300 h | 600 h | 900 h | 1200 h |
| Produkt von Beispiel 2 | 9 | 11 | 21 | 54 | - |
| - | 7 | 14 | 45 | 59 | - |

Tabelle 1b

Dehnbarkeitsmessungen und Yellowness Index von bei 150 °C ofengealterten Proben mit und ohne Stabilisator

| Additive | Dehnbarkeit der Probe (%) und Dauer der Hitzebelastung (h) | | | | |
|---|---|---|---|---|---|
| | 0 h | 0,5 h | 1 h | 1,5 h | 2 h |
| Produkt von Beispiel 2 | 50 | 51 | 14 | 7 | 6 |
| - | 33 | 7 | 7 | 3 | 3 |

| Additive | Yellowness Index und Dauer der Hitzebelastung (h) | | | | |
|---|---|---|---|---|---|
| | 0 h | 0,5 h | 1 h | 1,5 h | 2 h |
| Produkt von Beispiel 2 | 9 | 8 | 17 | 19 | 25 |
| - | 7 | 18 | 30 | 38 | 43 |

Beispiel 5

Lichtstabilität von Polypropylen.

Unstabilisiertes Polypropylenpulver (Hercules Profax® 6501) wird mit 0,2 Gew.% eines Additivs gemischt. Diese Mischungen werden anschliessend bei 182 °C 5 Minuten lang auf einem Mischwalzwerk plastifiziert. Anschliessend wird die stabilisierte Polypropylenfolie vom Walzwerk abgelöst und abkühlen gelassen. Die Folie wird in Stücke geschnitten und auf einer hydraulischen Presse bei 220 °C und 12 bar zu einer 0,13 mm dicken Probe verformt. Diese Probe wird ultraviolettem Licht bis zur Zersetzung ausgesetzt. Als Zersetzungszeitpunkt wird diejenige Anzahl von Stunden gewertet, bei der die ersten Zerfallserscheinungen (Risse, braune Ecken) auftreten. Die Messdaten sind in der folgenden Tabelle 2 dargestellt.

Tabelle 2

| Additiv | Zahl der Stunden bis zur Zersetzung der bestrahlten Proben |
|---|---|
| Produkt von Beispiel 1 | 500 |
| Produkt von Beispiel 2 | 450 |
| Produkt von Beispiel 3 | 400 |
| - | 200-300 |

Beispiel 6

Stabilisatorwirkung einer Kombination der erfindungsgemässen Verbindungen mit DSTDP in Polypropylen.

Unstabilisiertes Polypropylenpulver (Hercules Profax® 6501) wird mit 0,2 Gew.-% des jeweiligen Stabilisators gemischt. Zusätzlich werden Polypropylenproben hergestellt, die 0,1 Gew.-% einer erfindungsgemässen Verbindung und 0,3 Gew.-% Distearyl-β-thiodipropionat (DSTDP) enthalten.

Diese Mischungen werden anschliessend bei 182 °C 5 Minuten lang auf einem Mischwalzwerk plastifiziert. Anschliessend werden die Polypropylenfolien von der Walze entfernt und erkalten gelassen.

Die so vorbehandelten Proben werden zerkleinert und anschliessend 5 Minuten lang in einer hydraulischen Presse bei 220 °C und unter einem Druck von 12 bar zu Platten von 0,64 mm Dicke verformt. Diese Platten werden bei 150 °C in einem Umluftofen gealtert. Als Zersetzungszeitpunkt wird diejenige Anzahl von Stunden gewertet, nach der die Proben erste äusserliche Anzeichen eines Zerfalls (braune Ecken, Risse) zeigen.

Die Ergebnisse sind in der untenstehenden Tabelle aufgelistet.

| Stabilisator | Stabilisatormenge (Gew.%) | Zersetzungszeit (Stunden) |
|---|---|---|
| - | - | < 20 |
| DSTDP | 0,3 | < 20 |
| Produkt von Beispiel 1 | 0,2 | 310 |
| Produkt von Beispiel 1 + DSTDP | 0,1 + 0,3 | 570 |
| Produkt von Beispiel 2 | 0,2 | 520 |
| Produkt von Beispiel 2 + DSTDP | 0,1 + 0,3 | 1130 |
| Produkt von Beispiel 3 | 0,2 | 320 |
| Produkt von Beispiel 3 + DSTDP | 0,1 + 0,3 | 1300 |

**Patentansprüche**

1. Verbindungen der Formel I

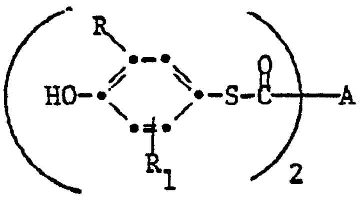

(I)

worin R und $R_1$ unabhängig voneinander Wasserstoff, $C_1$-$C_{12}$ Alkyl, $C_5$-$C_6$Cycloalkyl, Phenyl, durch $C_1$-$C_{12}$ Alkyl substituiertes Phenyl, $C_7$-$C_9$ Aralkyl oder durch $C_1$-$C_{12}$ Alkyl substituiertes $C_7$-$C_9$ Aralkyl bedeuten, und worin A $C_1$-$C_{10}$ Alkylen, $C_5$-$C_6$ Cycloalkylen, Arylen, Biphenylen oder hydroxysubstituiertes Arylen bedeutet.

2. Verbindungen der Formel I gemäss Anspruch 1, worin $R_1$ sich in ortho-Position zur Hydroxygruppe befindet.

3. Verbindungen der Formel I gemäss Anspruch 1, worin R und $R_1$ $C_1$-$C_8$ Alkyl bedeuten.

4. Verbindungen der Formel I gemäss Anspruch 1, worin R und $R_1$ tert.-Butyl bedeuten.

5. Verbindungen der Formel I gemäss Anspruch 1, worin A $C_4$-$C_8$ Alkylen oder Phenylen ist.

6. S,S'-Bis-(3,5-di-tert.-butyl-4-hydroxyphenyl)-dithioadipat gemäss Anspruch 1.

7. S,S'-Bis-(3,5-di-tert.-butyl-4-hydroxyphenyl)-dithiosebacat gemäss Anspruch 1.

8. S,S'-Bis-(3,5-di-tert.-butyl-4-hydroxyphenyl)-dithioterephthalat gemäss Anspruch 1.

9. Zusammensetzung enthaltend ein gegen oxidativen, thermischen oder strahlungsinduzierten Abbau empfindliches organisches Material und mindestens eine Verbindung der Formel I gemäss Anspruch 1.

10. Zusammensetzung gemäss Anspruch 9, worin das organische Material ein synthetisches Polymer ist.

11. Zusammensetzung gemäss Anspruch 10, worin das synthetische Polymer schlagfestes Polystyrol, ABS, Styrol-Butadien Kautschuk, Polyester oder ein Poly-α-olefin bedeutet.

12. Zusammensetzung gemäss Anspruch 9, worin das organische Material ein Mineralöl oder ein synthetisches Schmiermittel ist.

13. Verfahren zum Stabilisieren von organischem Material gegen oxidativen, thermischen oder strahlungsinduzierten Abbau, dadurch gekennzeichnet, dass man dem Material mindestens eine Verbindung der Formel I gemäss Anspruch 1 zusetzt.

14. Verwendung von Verbindungen der Formel I gemäss Anspruch 1 als Stabilisatoren für organisches Material gegen dessen Schädigung durch Einwirkung von Sauerstoff, Wärme, Licht oder energiereiche Strahlung.

**Claims**

1. A compound of the formula I

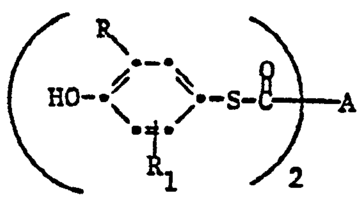

(I)

wherein R and $R_1$ are each independently hydrogen, alkyl of 1 to 12 carbon atoms, cycloalkyl of 5 to 6 carbon atoms, phenyl, phenyl substituted by alkyl of 1 to 12 carbon atoms, or aralkyl of 7 to 9 carbon atoms or said aralkyl substituted by alkyl of 1 to 12 carbon atoms ; and A is alkylene of 1 to 10 carbon atoms, cycloalkylene of 5 to 6 carbon atoms, arylene, biphenylene or hydroxy-substituted arylene.

2. A compound of formula I according to claim 1, wherein $R_1$ is in the ortho-position to the hydroxyl group.

3. A compound of formula I according to claim 1, wherein R and $R_1$ are alkyl of 1 to 8 carbon atoms.

4. A compound of formula I according to claim 1, wherein R and $R_1$ are tert-butyl.

5. A compound of formula I according to claim 1, wherein A is alkylene of 4 to 8 carbon atoms or phenylene.

6. S,S'-Bis(3,5-di-tert-butyl-4-hydroxyphenyl) dithioadipate according to claim 1.

7. S,S'-Bis(3,5-di-tert-butyl-4-hydroxyphenyl) dithiosebacate according to claim 1.

8. S,S'-Bis(3,5-di-tert-butyl-4-hydroxyphenyl) dithioterephthalate according to claim 1.

9. A composition containing an organic material subject to oxidative, thermal or radiation-induced degradation and at least one compound of formula I according to claim 1.

10. A composition according to claim 9, wherein the organic material is a synthetic polymer.

11. A composition according to claim 10, wherein said synthetic polymer is selected from the group

consisting of impact-resistant polystyrene, acrylonitrile/butadiene/styrene, styrene/butadiene rubber, polyesters and poly-alpha-olefins.

12. A composition according to claim 9, wherein the organic material is a mineral oil or a synthetic lubricant.

13. A method of stabilizing an organic material against oxidative, thermal or radiation-induced degradation, which comprises incorporating into said organic material at least one compound of formula I according to claim 1.

14. Use of a compound of formula I according to claim 1 for stabilizing organic material against the action of oxygen, heat, light or high-energy radiation.

**Revendications**

1. Composés répondant à la formule I :

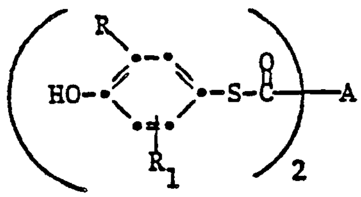

(I)

dans laquelle R et $R_1$ représentent chacun, indépendamment l'un de l'autre, l'hydrogène, un alkyle en $C_1$-$C_{12}$, un cycloalkyle en $C_5$ ou $C_6$, un phényle, un phényle porteur d'un alkyle en $C_1$-$C_{12}$, un aralkyle en $C_7$-$C_9$ ou un aralkyle en $C_7$-$C_9$ porteur d'un alkyle en $C_1$-$C_{12}$, et A représente un alkylène en $C_1$-$C_{10}$, un cycloalkylène en $C_5$-$C_6$, un arylène, un biphénylylène ou un arylène porteur d'un hydroxy.

2. Composés de formule I selon la revendication 1 dans lesquels $R_1$ se trouve en position ortho par rapport au radical hydroxy.

3. Composés de formule I selon la revendication 1 dans lesquels R et $R_1$ représentent chacun un alkyle en $C_1$-$C_8$.

4. Composés de formule I selon la revendication I dans lesquels R et $R_1$ représentent chacun un radical tert-butyle.

5. Composés de formule I selon la revendication 1 dans lesquels A représente un alkylène en $C_4$-$C_8$ ou un phénylène.

6. Composé selon la revendication 1, en l'espèce le dithio-adipate de S,S'-bis-(di-tert-butyl-3,5 hydroxy-4 phényle).

7. Composé selon la revendication 1, en l'espèce le dithiosébaçate de S,S'-bis-(di-tert-butyl-3,5 hydroxy-4 phényle).

8. Composé selon la revendication 1, en l'espèce le dithiotéréphtalate de S,S'-bis-(di-tert-butyl-3,5 hydroxy-4 phényle).

9. Composition contenant une matière organique sensible à la dégradation par oxydation, par la chaleur ou sous l'effet d'un rayonnement, et au moins un composé de formule I selon la revendication 1.

10. Composition selon la revendication 9 dans laquelle la matière organique est un polymère synthétique.

11. Composition selon la revendication 10 dans laquelle le polymère synthétique est un polystyrène de haute résilience, l'ADS, un caoutchouc styrène/butadiène, un polyester ou une poly-α-oléfine.

12. Composition selon la revendication 9 dans laquelle la matière organique est une huile minérale ou un lubrifiant synthétique.

13. Procédé pour stabiliser une matière organique contre la dégradation par oxydation, par la chaleur ou sous l'effet d'un rayonnement, procédé caractérisé en ce qu'on ajoute à la matière au moins un composé de formule I selon la revendication 1.

14. Application des composés de formule I selon la revendication 1 comme stabilisants pour des matières organiques contre les dommages que pourraient leur occasionner l'action de l'oxygène, la chaleur, la lumière ou un rayonnement de haute énergie.